# EUROPEAN PATENT APPLICATION

(11) **EP 1 870 422 A1**
(43) Date of publication of application: **26.12.2007**
(21) Application number: 06012684.4
(22) Date of filing: 20.06.2006
(51) Int. Cl.: C07K 16/28, A61P 37/00, C12N 15/13, A61K 39/00

(54) **Means for the treatment of diseases characterized by an excessive immune reaction**

(71) Applicant: SuppreMol GmbH, 82152 Martinsried (DE)
(72) Inventor: Jacob, Uwe, 80339 Munich (DE)
(74) Representative: Bohmann, Armin K.

(57) **Abstract**

The present invention is related to an agent that binds FcgammaRIIb with higher affinity than FcgammaRIIa but does not interfere with the function of the FcgammaRIIb to bind to the Fc-fragment of immune globulins.

SEQUENCE LISTING

<110> SuppreMol GmbH

<120> Means for the treatment of diseases characterized by an excessive immune
reation

<130> S 10032 EP

<160> 2

<170> PatentIn version 3.1

<210> 1
<211> 181
<212> PRT
<213> Homo sapiens

<220>
<221> MISC_FEATURE
<223> Extracellular domain of FcgammaRIIb

<400> 1

Ala Gly Thr Pro Ala Ala Pro Pro Lys Ala Val Leu Lys Leu Glu Pro
1               5                   10                  15

Gln Trp Ile Asn Val Leu Gln Glu Asp Ser Val Thr Leu Thr Cys Arg
            20                  25                  30

Gly Thr His Ser Pro Glu Ser Asp Ser Ile Gln Trp Phe His Asn Gly
        35                  40                  45

Asn Leu Ile Pro Thr His Thr Gln Pro Ser Tyr Arg Phe Lys Ala Asn
    50                  55                  60

Asn Asn Asp Ser Gly Glu Tyr Thr Cys Gln Thr Gly Gln Thr Ser Leu
65                  70                  75                  80

Ser Asp Pro Val His Leu Thr Val Leu Ser Glu Trp Leu Val Leu Gln
                85                  90                  95

Thr Pro His Leu Glu Phe Gln Glu Gly Glu Thr Ile Val Leu Arg Cys
            100                 105                 110

His Ser Trp Lys Asp Lys Pro Leu Val Lys Val Thr Phe Phe Gln Asn
        115                 120                 125

Gly Lys Ser Lys Lys Phe Ser Arg Ser Asp Pro Asn Phe Ser Ile Pro
    130                 135                 140

Gln Ala Asn His Ser His Ser Gly Asp Tyr His Cys Thr Gly Asn Ile
145                 150                 155                 160

Gly Tyr Thr Leu Tyr Ser Ser Lys Pro Val Thr Ile Thr Val Gln Ala
                165                 170                 175

Pro Ser Ser Ser Pro
            180

<210>  2
<211>  310
<212>  PRT
<213>  Homo sapiens

<220>
<221>  MISC_FEATURE
<223>  FcgammaRIIb

<400>  2

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Ala Pro Pro
        35                  40                  45

Lys Ala Val Leu Lys Leu Glu Pro Gln Trp Ile Asn Val Leu Gln Glu
    50                  55                  60

Asp Ser Val Thr Leu Thr Cys Arg Gly Thr His Ser Pro Glu Ser Asp
65                  70                  75                  80

Ser Ile Gln Trp Phe His Asn Gly Asn Leu Ile Pro Thr His Thr Gln
                85                  90                  95

Pro Ser Tyr Arg Phe Lys Ala Asn Asn Asn Asp Ser Gly Glu Tyr Thr
            100                 105                 110

Cys Gln Thr Gly Gln Thr Ser Leu Ser Asp Pro Val His Leu Thr Val
        115                 120                 125

Leu Ser Glu Trp Leu Val Leu Gln Thr Pro His Leu Glu Phe Gln Glu
    130                 135                 140

Gly Glu Thr Ile Val Leu Arg Cys His Ser Trp Lys Asp Lys Pro Leu
145                 150                 155                 160

Val Lys Val Thr Phe Phe Gln Asn Gly Lys Ser Lys Lys Phe Ser Arg

                165                 170                 175

Ser Asp Pro Asn Phe Ser Ile Pro Gln Ala Asn His Ser His Ser Gly
            180                 185                 190

Asp Tyr His Cys Thr Gly Asn Ile Gly Tyr Thr Leu Tyr Ser Ser Lys
        195                 200                 205

Pro Val Thr Ile Thr Val Gln Ala Pro Ser Ser Ser Pro Met Gly Ile
    210                 215                 220

Ile Val Ala Val Val Thr Gly Ile Ala Val Ala Ala Ile Val Ala Ala
225                 230                 235                 240

Val Val Ala Leu Ile Tyr Cys Arg Lys Lys Arg Ile Ser Ala Leu Pro
                245                 250                 255

Gly Tyr Pro Glu Cys Arg Glu Met Gly Glu Thr Leu Pro Glu Lys Pro
            260                 265                 270

Ala Asn Pro Thr Asn Pro Asp Glu Ala Asp Lys Val Gly Ala Glu Asn
        275                 280                 285

Thr Ile Thr Tyr Ser Leu Leu Met His Pro Asp Ala Leu Glu Glu Pro
    290                 295                 300

Asp Asp Gln Asn Arg Ile
305                 310

## Description

The present invention is related to means for the treatment of diseases characterized by an excessive immune reaction.

In recent years, medical professionals have become aware that diseases with an excessive immune reaction occur more frequently. This kind of disease comprise, among others, autoimmune diseases and various allergies as well as asthma all of which are characterized by an overactive immune system.

More specifically, autoimmune diseases result when the immune system mistakes self-tissue for foreign and mounts an inappropriate attack. The autoimmune disease can affect different tissues and organs of the body, is hardly curable, and is often chronic requiring lifelong care and monitoring. The underlying mechanisms are hardly understood and appropriate medication is rare. While the initiation of the autoimmune process is believed to be a corruption of T cell regulation, the tissue damage and perpetuation of the diseases are often due to immune complexes. Because of this, Fc receptors, as major initiators of immune complex mediated inflammation, receive an increasing attention as potential therapeutic target for the treatment of autoimmune disorders.

As a critical link between the humoral and cellular immunity receptors for the Fc portion of IgG antibodies (FcγRs) play a pivotal role in a number of important immunological mechanisms such as phagocytosis, clearance of immune complexes and antibody dependent cellular cytotoxicity (ADCC) (Gessner et al. 1998, Ravetch and Bolland 2001). Moreover, they indirectly stimulate the release of mediators and antigen re-presentation to APCs, thereby promoting the pro-inflammatory feed-back loop in autoimmune diseases (Hogarth 2002). The direct involvement of the Fc-receptors in the progression of autoimmune disease has thereby impressively been shown in mice models of Rheumatoide Arthiritis (CIA), Multiple Sclerosis (EAE) and Systemic Lupus Erythematodis (NZ-B/W) that were carried out with mice interbreed on an Fc-receptor deficient back ground (Abdul-Majid 2002, van Lent 2001).

In humans the FcγR family comprises the distinct sub-classes FcγRI, FcγRIII and FcγRIIa, which exist as oligomeric complexes involved in cellular activation mediated by their immunoreceptor tyrosine-based activation motif (ITAM) in their cytoplasmic domain. In contrast the FcγRIIb in humans is a monomeric receptor containing an inhibitory (ITIM) signalling motif, which limits effective signalling (Gessner 1998). The murine counterpart to FcγRIIb is FcγRII which is composed of two immunoglobulin-like extracellular domains, a transmembrane domain and a cytoplasmic tail. At protein level four isoforms generated by alternative splicing have been described, whereas at the gene level a polymorphism in the form of the Ly-17.1 - Ly17.2 alloantigenic system was revealed (Hibbs 1985, Slingsby 1997).

The inhibitory IgG binding Fc receptor, FcγRIIb plays an indispensable role in the suppression of antibody-mediated autoimmunity. In contrast, the activating-type Fc receptors (FcRs) are essential for the development of these diseases (Clynes, 1999; Nakamura, 2005).

The activating receptor FcγRIII and the inhibitory receptor FcγRIIb are usually found coexpressed on the cellular surface and bind IgG with comparable affinity and specificity. The co-engagement of both receptors determined by the ratio of expression results in balanced effector cell responses. A common mechanism is the hetero-aggregation of an ITAM-containing with an ITIM-containing receptor, which leads to the inhibition of ITAM-triggered calcium mobilization and cellular proliferation Ravetch, 2001. In B-cells that exclusively express the FcγRIIb the ITAM is associated with the cytoplasmic domain of the specific B-cell receptor (Fridman 1993).

Nevertheless, for Fc-receptor directed therapy of autoimmune diseases selective (ant)agonists are required in order to investigate their potential to influence the homeostasis of activating and inactivating Fc-receptors. The prior art describes several approaches, which, however, are not suitable to provide for an efficient treatment of diseases characterised by an excessive immune response, particularly an excessive immune response where IgG and IgE antibodies are generated by the organism suffering from such disease or being at risk to develop such disease. A suitable antagonist has been described by Sondermann et al. (Sondermann 1999) which is a homogenous preparation of soluble FcγRIIb (sFcγRIIb) expressed in E. coli. An agonist is described by Samuelson et. al. (Samuelson 2001) which is a specific anti Ly-17.1 (mouse FγRII) antibody (antiFcgRII-Ab). A still further agent which is said to be effective in the treatment of this kind of disease, is the antibody against the human FcγRIIb described in international patent application WO 2004/16750 and WO 2005/051999 .

The problem underlying the present invention is to provide means for the treatment of diseases characterized by an excessive immune response.

In a first aspect the problem underlying the present invention is solved by an agent that binds FcgammaRIIb with higher affinity than FcgammaRIIa but does not interfere with the function of the FcgammaRIIb to bind to the Fc-fragment of immune globulins.

In an embodiment the binding of immune complexes to FcgammaRIIB is not inhibited.

In an embodiment the binding site of the agent recognizes at least one amino acid residue that is different between FcgammaRIIa and Fcgamma RIIB, preferably two, more preferably three of such amino acid residues, whereby such amino acid(s) is/are not involved in Fc-fragment binding.

In a preferred embodiment such amino acid(s) is/are present in the N-terminal region or the N-terminal domain or the C-terminal linker region of FcgammaRIIb.

In a further preferred embodiment the amino acids are selected from the group comprising amino acids 17, 32, 33, 35, 109 and 176 of the amino acid sequence of SEQ.ID.No 1, more preferably of human FcgammaRIIb.

In an embodiment the amino acids are amino acids 32 to 35 of SEQ.ID.No.1.

In an embodiment the binding affinity to FcgammaRIIb is higher than the binding affinity to FcgammaRIIa, preferably at least 2fold, more preferably at least 4fold, more preferably at least 10fold, more preferably at least 100fold, more preferably at least 1000fold, more preferably at least 10.000fold and most preferably 100.000 fold higher.

In an embodiment the agent is selected from the group comprising antibodies, bispecific antibodies, trispecific antibodies, anticalines, aptamers and spiegelmers.

In a preferred embodiment the agent is an antibody, preferably a monoclonal antibody and more preferably a humanized antibody.

In a further preferred embodiment the agent is a human antibody.

In an embodiment the agent is a F(ab')₂ fragment.

In an embodiment the agent is a F(ab) fragment.

In an embodiment the agent is a single chain antibody.

In an embodiment the agent is an antibody of the IgG class.

In an embodiment the agent is an antibody of the IgE type.

In a second aspect the problem underlying the present invention is solved by a nucleic acid coding the agent and more preferably an antibody according to any embodiment of the first aspect.

In a third aspect the problem underlying the present invention is solved by a vector, preferably an expression vector, comprising a nucleic acid according to the second aspect.

In a fourth aspect the problem underlying the present invention is solved by a cell, preferably an isolated cell, comprising a nucleic acid according to the second aspect or a vector according to the third aspect.

In a fifth aspect the problem underlying the present invention is solved by a pharmaceutical composition comprising an agent, preferably an antibody or a nucleic acid, according to any aspect of the present invention, whereby the composition preferably contains a pharmaceutically acceptable vehicle.

In a sixth aspect the problem underlying the present invention is solved by the use of an agent, preferably an antibody, according to any of the aspects for the manufacture of a medicament.

In a seventh aspect the problem underlying the present invention is solved by the use of a nucleic acid according to any of the aspects for the manufacture of a medicament.

In an embodiment of the sixth and seventh aspect the medicament is for the treatment of an autoimmune disease.

In an embodiment of the sixth and seventh aspect the medicament is for the treatment of a B-cell lymphoma.

In an embodiment of the sixth and seventh aspect the autoimmune disease is selected from the group comprising inflammatory responses such as inflammatory skin diseases including psoriasis and dermatitis inclduing atopic dermatitis; systemic scleroderma and sclerosis; responses associated with inflammatory bowel disease including Crohn's disease and ulcerative colitis; respiratory distress syndrome including adult respiratory distress syndrome; ARDS; dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus including Type I diabetes mellitus or insulin dependent diabetes mellitis; multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia including cryoglobinemia or Coombs positive anemia ; myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) and autoimmune thrombocytopenia.

In an embodiment of the sixth and the seventh aspect the agent is an antibody of the IgG type as defined in any of the proceeding claims.

In an embodiment of the sixth and the seventh aspect the disease is selected from the group comprising allergic diseases and asthma.

In a preferred embodiment of the sixth and the seventh aspect the agent is an antibody of the IgE type.

In an eighth aspect the problem underlying the present invention is solved by a method for producing an antibody that specifically binds FcgammaRIIb but does not block a function of the FcgammaRIIb, more preferably of an antibody as described in any embodiment of the first aspect, whereby the method comprises the following steps:
(a) immunizing a mouse with a peptide containing amino acids 32-35 (RGTH) of FcgammaRIIb;
(b) optionally booster immunizing said mouse for a time sufficient to elicit an immune response;
(c) producing hybridoma cell lines from spleen cells of said mouse; and
(d) screening said hybridoma cell lines for one or more hybridoma cell lines that produce antibodies that bind FcgammaRIIb but does not block a function of the FcgammaRIIb.

The problem is solved by the subject matter of the independent claims. Particularly preferred embodiments may be taken from the dependent claims.

The present invention is based on the surprising finding of the present inventors that agents which are able to bind to FcgammaRIIb with higher affinity than to FcgammaRIIa and the binding of which does not interfere with the binding of the Fc-moiety of immune globulins, are suitable to act as means for the suppression of an excessive immune response.

Agents such as antibodies which bind to Fcgamma RIIb with higher affinity than to FcgammaRIIa have, in principle, already been described in the prior art such as in WO 2004/16750 and WO 2005/051999. However, these agents of the prior art interact with the binding site of the FcgammaRIIb in a way such that the inhibiting activity of the FcgammaRIIb within immune cells is not mediated. Insofar, these antibodies are blocking antibodies, i.e. antibodies blocking the desired activity of FcgammaIIb. In accordance therewith the present inventors were able to show that these FcgammaRIIb blocking antibodies do not inhibit the immune reaction as desired; rather a contra-productive immune stimulation is observed when higher concentrations of antibodies having these characteristic are applied in animal models of autoimmune diseases.

Without wishing to be bound by any theory, the present inventors currently assumes that one possibility of the immune stimulatory effect of these agents of the prior art is the blockade of FcgammaRIIb on B-cells. FcgammaRIIb is the only Fc-receptor expressed by B-cells and B-cells depend on the functional activity of this receptor to limit their excitation state, i.e.production level of antibodies. Such blockade of FcgammaRIIb by these agents of the prior art interferes with the functional activity of this receptor and the consequence is an even increased state of activation and antibody production by said B cells.

The problem of this contra-productive effect is thus solved in accordance with the present invention by an agent that recognizes FcγRIIb with higher affinity than FcγRIIa but does not interfere with immune complex recognition and immune complex binding, respectively, of the receptor, i.e. FcgammaRIIb. In other words, the characteristics of the agents of the present invention can be linked to the following requirements. The low affinity to FcgammaRIIa, which is significantly lower compared to the affinity to FcgammaRIIb, makes sure that the agent does not stimmulate other immune cells through FcgammaRIIa which is known as being part of the stimulatory branch of the Fcgamma mediated immune response. The increased affinity to FcgammaRIIb allows for the selective or highly specific interaction of the agent with FcgammaRIIb which is known as being the inhibitory branch of the Fcgamma mediated immune response. By not interfering, more preferably not inhibiting the binding of the Fc-fragment of immune globulins to FcgammaRIIb, the Fc-fragment associated effects such as cross-linking of FcgammaRIIb and B-cell receptors by immune complexes can still be observed and result in an overall inhibitory effect of the agent on B-cells. According to the present invention a beneficial effect due to decreased B-cell activation and antibody production in autoimmune disease and therefore the progression of the diseases can be observed.

The same arguments and strategy, respectively, apply for the inhibition of allergic responses that involve FcepsilonRI signalling. For example this receptor is expressed on Mast cells and crosslinking with FcγRIIb leads to effective inhibition of the cell. And again using an IgE antibody that binds FcγRIIb with higher affinity than FcγRIIa and which does not interfere with the inhibitory activity of FcγRIIb in immune complex recognition, would clearly be an advantage over compareable IgE antibodies that block FcγRIIb as disclosed in WO 2004/16750 and WO 2005/051999.

In connection with the present invention, the feature of the agent of the present invention of not binding, or at least not binding in a significant manner, to the FcgammaRIIa which is responsible for the activation and promotion of many immune cells, preferably means that the binding affinity is, compared to the binding characteristics to the FcgammaRIIb, reduced by at least a factor, whereby such factor is selected from the group of 2; 10; 100; 1000; 10,000 and 100,000.

It is within the present invention that the binding of the agent in accordance with the present invention is mediated by a binding site present on said agent. Such binding site mediates either directly or indirectly the binding of the agent to an interaction partner, preferably the binding of the agent to FcgammaRIIb. More specifically, the binding site of the agent of the present invention is interacting with a target site on the molecule with which the agent is interacting or is to interact. The molecule, with which the agent is interacting or is to interact, is preferably FcgammaRIIb, more preferably mammalian FcgammaRIIB and even more preferably human FcgammaRIIb.

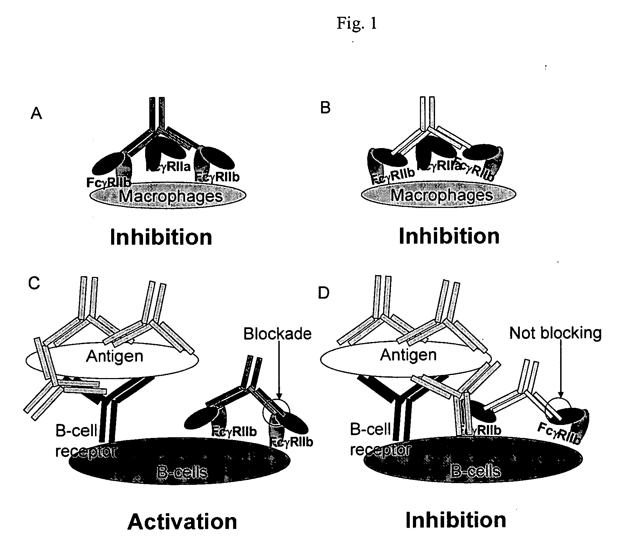

The target site of FcgammaRIIb recognized by the agent according to the present invention is preferably either the N-terminal domain of FcgammaRIIb, preferably the extracellular domain, or the flexible linker region that connects the extracellular domain thereof with the plasma membrane. More preferably, such agent does not interact with the C-terminal domain of FcgammRIIb that is involved in Fc-fragment binding in immune complex recognition. More specific regions and even individual amino acids or cluster of amino acids acting as target sites on FcgammaRIIb, can be determined by a sequence alignment so as to make sure that such amino acid(s) are not represent in FcgammaRIIa providing for one of the features of the agents according to the present invention. In an alternative, preferably additional step, crystal structures are used to identify those parts of the FcgammaRIIb which are not involved in the interaction between Fc-fragments and FcgammaRIIb. Such data can, e.g., be taken from Sondermann (Sondermann 2000). Particularly preferred target sites are insofar those defined by amino acid positions 17, 32, 34, 35, 109 and 112 of the amino acid sequence of human FcgammaRIIb and SEQ.ID:No. 1, respectively, or those depticed in Fig. 1 and positions corresponding thereto. It will be acknowledged by the ones skilled in the art that target sites can also be part of bigger target sites. A particularly preferred target site is the region corresponding to amino acid positions 32-35 of SEQ.ID.NO. 1 because the differing amino acid residues cluster in a single epitope (nearby in space) of the FcγRIIb and are not involved in Fc binding by FcgammaRIIb. In further embodiments, the bigger target site comprises of from 5, more preferably from about 8, more preferably from about 10 to about 30, more preferably to about 20, more preferably to about 18, more preferably to about 15 amino acids, whereby preferably at least one of said amino acids is contained therein and even more preferably provides for the required specificity to discriminate between the binding of the agent of the present invention to FcgammaRIIb and FcgammaRIIa.

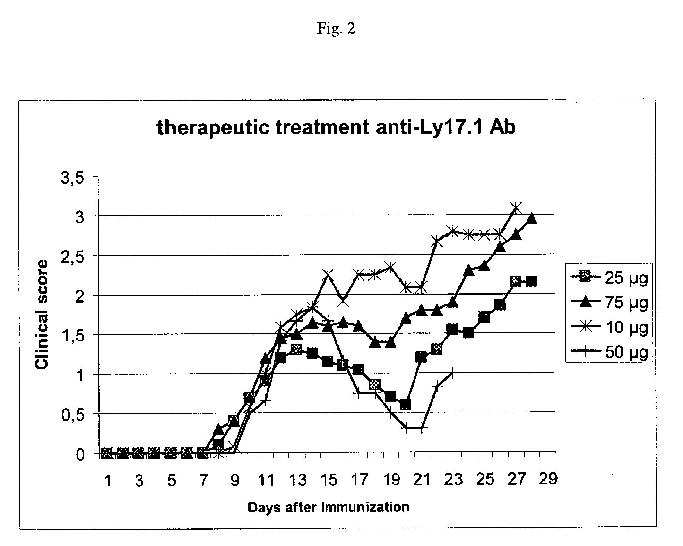

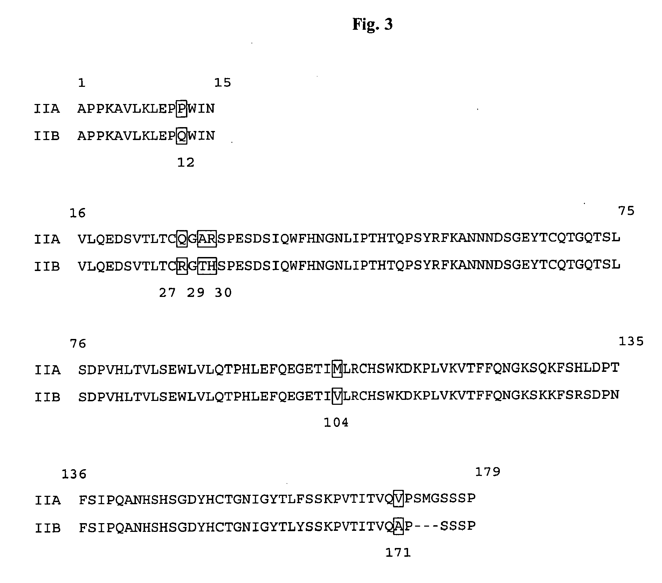

That the balancing of these characteristics allows for a beneficial effect on diseases characterised by an excessive immune response may be taken from an experiment in which the antibody anti-Ly17.1 is used in a dose dependent manner. Said experiment is described in more detail in the example part of this specification. Briefly, using the anti-Ly17.1 antibody at different concentrations mimics the use of antibodies having some or all of the characteristics of the agents of the prior art and more specifically the antibodies described in WO 2004/16750 and WO 2005/051999. Anti- Ly17.1 binds to the inhibitory FcγRII in mice without any crossreactivity towards other Fc-receptors, i.e. interferes with the binding of Fc fragments to FcgammaRII and blocks at the same time the natural inhibitory function of this receptor. More specifically using the anti-Ly17.1 antibody at a concentration of 25 µg and 50 µg / injected mouse results in a significant improvement in clinical score in the animal model used. This can be explained by the fact that blocking anti-FcR-antibodies like anti-Ly17.1 carry two binding sites for the respective Fc-receptor on their Fab arms but can bind a third prefereably high affinity Fc-receptor via their Fc-fragment. This trimeric interaction results in an inhibitory signal to immune cells that do carry such a third Fc-receptor which is not the case on B-cells. If such an antibody is now given at slightly higher concentrations, all positions for trimeric interactions are saturated and dimeric binding on B-cells which express only the inhibitory Fc-receptor, is the consequence. Therefore as seen in the experiment (Fig. 2) the use of higher concentrations, results in a drastic worsening of the clinical score. This result is a reflection of the blockade of the inihibiting FcgammaRIIb by the antibody anti-Ly17.1 on antibody producing B cells. The experiment therefore shows that antibodies described in the prior art such as in WO 2004/16750 and WO 2005/051999 are not suitable for the treatment of autoimmune diseases. Instead the agents according to the present invention are suitable for such purpose which are able to inhibit immune cells by trimeric binding but do not block the vital function of the inhibitory Fc-receptor if it comes to FcγRIIb binding on B-cells as also illustrated in Fig. 3.

In the light of the present disclosure, it is possible for the one skilled in the art to generate the kind of agent claimed and to use such agent for the purpose as outlined herein.

More specifically, the agents which have the characteristics as outlined herein are preferably selected from the group comprising antibodies, high affinity binding peptides, anticalines, apatmers and spiegelmers. More specifically, knowing the design principles and functional requirements as outlined herein the target sites which can be used for the generation of said agents are those outlined above.

It is within the present invention that the agents of the present invention are individually or in any combination used for the manufacture of a medicament. More preferably such medicament is for the treatment and/or prevention of a disease. Even more preferably such disease is an autoimmune disease. An autoimmune disease as preferably used herein is a non-malignant disease or disorder arising from and directed against an individual's own tissues. Examples of autoimmune diseases or disorders include, but are not limited to, inflammatory responses such as inflammatory skin diseases including psoriasis and dermatitis (for example, atopic dermatitis); systemic scleroderma and sclerosis; responses associated with inflammatory bowel disease (such as Crohn's disease and ulcerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (e.g. Type I diabetes mellitus or insulin dependent diabetes mellitis); multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia) ; myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia. However it is also within the present invention that the medicament may be used for the treatment and/or prevention of B cell lymphoma.

A particularly preferred agent is an antibody having the characteristics of the agent in accordance with the present invention. The generation of antibodies and any of the derivatives thereof disclosed herein having distinct characteristics is known to the ones skilled in the art and, e.g., described in Harlow E. et al., Antibodies: A Laboratory Manual, Cold Spring Harbor Laboratory Press, 1988.

The terms "antibody" and immunoglobulin are used interchangeably in the broadest sense and include monoclonal antibodies, e.g., full length or intact monoclonal antibodies, polyclonal antibodies, multispecific antibodies, e.g., bispecific antibodies so long as they exhibit the desired biological activity, and may also include certain antibody fragments (as described in greater detail herein), such as, for example, antigen binding polypeptides which polypeptides may be fragments of an antibody. In one embodiment, antibodies and immunoglobulins of the present invention have reduced (fewer) disulfide linkages. In one embodiment, antibodies and immunoglobulins of the invention comprise a hinge region in which at least one cysteine residue is rendered incapable of forming a disulfide linkage, wherein the disulfide linkage is preferably intermolecular, preferably between two heavy chains. A hinge cysteine can be rendered incapable of forming a disulfide linkage by any of a variety of suitable methods known in the art, some of which are described herein, including but not limited to deletion of the cysteine residue or substitution of the cysteine with another amino acid.

An antibody may be part of a larger fusion molecule, formed by covalent or non-covalent association of the antibody with one or more other protein or peptide. The terms "full length antibody," "intact antibody" and "whole antibody" are used herein interchangeably, to refer to an antibody in its substantially intact form, and not antibody fragments as defined below. The terms particularly refer to an antibody with heavy chains contains Fc regions. An antibody variant of the invention can be a full length antibody. A full length antibody can be human, humanized, chimeric, and/or affinity matured.

"Antibody fragments" preferably comprise only a portion of an intact antibody, where the portion retains at least one, and may retain most or all, of the functions normally associated with that portion when present in an intact antibody. An antibody fragment of the invention may preferably comprise a sufficient portion of the constant region to permit dimerization (or multimerization) of heavy chains that have reduced disulfide linkage capability, for example where at least one of the hinge cysteines normally involved in inter-heavy chain disulfide linkage is altered as described herein. In one embodiment, an antibody fragment comprises an antigen binding site or variable domains of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function, and/or complement binding (for example, where the antibody has a glycosylation profile necessary for ADCC function or complement binding). Examples of antibody fragments include linear antibodies; single-chain antibody molecules; and multispecific antibodies formed from antibody fragments. More preferably an antibody fragment in accordance with the present invention is a fragment of an antibody which exhibits the characteristics of the agent in accordance with the present invention.

The term "chimeric" antibodies preferably refers to antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (See, for example, U.S. Patent No. 4,816,567 and Morrison etal., 1984, Proc. Natl. Acad. Sci. USA 81:6851-6855).

"Humanized" forms of non-human such as, for example, murine antibodies are preferably chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non- human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non- human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et ai, Nature 321:522-525 (1986); Riechmann et al, Nature 332:323-329 (1988); and Presta, Curr. Op. Struct. Biol. 2:593-596 (1992).

A "human antibody" as preferably used herein is an antibody that possesses an amino acid sequence corresponding to that of an antibody produced by a human and/or has been made using any of the techniques for making human antibodies disclosed herein. This definition specifically excludes a humanized antibody that comprises non-human antigen-binding residues.

In a further preferred embodiment the antibody is an isolated antibody. An "isolated" antibody is preferably one that has been identified and separated and/or recovered from a component of its natural environment. Contaminant components of its natural environment are materials that would interfere with diagnostic or therapeutic uses for the antibody, and may include enzymes, hormones, and other proteinaceous or nonproteinaceous solutes. In some embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

The term "monoclonal antibody" as preferably used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., 1975, Nature 256:495, or may be made by recombinant DNA methods (see, for example, U.S. Patent No. 4,816,567). The "monoclonal antibodies" may also be isolated from phage antibody libraries using the techniques described in Clackson et al, 1991, Nature 352:624-628 and Marks et al, 1991, /. Mol. Biol. 222:581-597, for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., 1984, Proc. Natl. Acad. ScL USA 81:6851-6855).

The technical teaching on how to generate antibodies which can discriminate between such closely related proteins like FcgammaIIb and FcgammaIIa is, for example described in international patent application WO 2005/051999. A further method for the generation of this kind of agents, particularly if such agent is an antibody, is described in WO 2004/16750. Said method involves the use of transgenic animals, more specifically transgenic mice. The transgenic mice are transgenic for the human FcgammaRIIa and are immunized using human FcgammaRIIb. Because of self-tolerance, the thus immunized transgenic animals will only produce antibodies which are directed agains FcgammaRIIb.

It is also within the present invention that the antibody of the present invention is generated by first generating an antibody having the characteristics as disclosed herein, and subsequently transferring the respective binding site thereof to a different framework, more preferably a different antibody framework. A particularly preferred framework is the IgE framework. The transfer of such binding site to an IgE framework allows for the treatment of IgE mediated diseases and more specifically allergies and asthma. As preferably used herein IgE mediated diseases are diseases or conditions which involve, preferably in a causal manner, an abundant IgE reaction of the organism suffering from such IgE mediated diseases or, at least, being at risk of suffering therefrom.

A further class of agents which can be used in accordance with the present invention are high-affinity binding peptides. It will be acknowledged by the ones skilled in the art that antibody fragments as described herein, may form such high-affinity binding peptides. However, this kind of compounds can also be generated in a different manner as outlined in the following.

In general, high-affinity binding peptides as used in a preferred embodiment are peptides which bind to a target molecule and target site, respectively, as defined hererin. Such peptides may be generated by using methods according to the state of the art such as, e.g., phage display. Basically, a library of peptides is generated, such as in the form of phages, and this kind of library is contacted with the target molecule. Those peptides binding to the target molecule are subsequently removed, preferably as a complex with the target molecule, from the respective reaction. It is known to the one skilled in the art that the binding characteristics, at least to a certain extend, depend on the particularly realized experimental set-up such as the salt concentration and the like. After separating those peptides binding to the target molecule with a higher affinity or a bigger force, from the non-binding members of the library, and optionally also after removal of the target molecule from the complex of target molecule and peptide, the respective peptide(s) may subsequently be characterised. Prior to the characterisation optionally an amplification step is realized such as, e. g. by propagating the peptide coding phages. The characterisation preferably comprises the sequencing of the target binding peptides. Basically, the high-affinity binding peptides are not limited in their lengths, however, preferably peptides having a lengths from about 8 to 20 amino acids are preferably obtained in the respective methods. The size of the libraries may be about 10² to 10¹⁸, preferably 10⁸ to 10¹⁵ different peptides, however, is not limited thereto.

A further class of agents which can be used in accordance with the present invention are anticalines which are, in principle, known to the ones skilled in the art. Basically, anticalines are a particular form of high-affinity target binding polypeptides which are generally accepted as an alternative to antibodies. Similar to antibodies, anticalines can be identified so as to bind to any target molecule. Anticalines are, among others, described in German patent application DE 197 42 706.

A further class of agents which can be used in accordance with the present invention are aptamers which, in principle, are known to the ones skilled in the art. Aptamers are D-nucleic acids which are either single stranded or double stranded and which specifically interact with a target molecule. The manufacture or selection of aptamers is, e. g., described in European patent EP 0 533 838. Basically the following steps are realized. First, a mixture of nucleic acids, i. e. potential aptamers, is provided whereby each nucleic acid typically comprises a segment of several, preferably at least eight subsequent randomised nucleotides. This mixture is subsequently contacted with the target molecule whereby the nucleic acid(s) bind to the target molecule, such as based on an increased affinity towards the target or with a bigger force thereto, compared to the candidate mixture. The binding nucleic acid(s) are/is subsequently separated from the remainder of the mixture. Optionally, the thus obtained nucleic acid(s) is amplified using, e. g., polymerase chain reaction. These steps may be repeated several times giving at the end a mixture having an increased ratio of nucleic acids specifically binding to the target from which the final binding nucleic acid is then optionally selected. These specifically binding nucleic acid(s) are referred to as aptamers. It is obvious that at any stage of the method for the generation or identification of the aptamers samples of the mixture of individual nucleic acids may be taken to determine the sequence thereof using standard techniques. It is within the present invention that the aptamers may be stabilized such as, e. g., by introducing defined chemical groups which are known to the one skilled in the art of generating aptamers. Such modification may for example reside in the introduction of an amino group at the 2'-position of the sugar moiety of the nucleotides. Aptamers are currently used as therapeutical agens.

A further class of agents which can be used in accordance with the present invention arespiegelmers which are, in principle, known to the ones skilled in the art. The generation or manufacture of spiegelmers which may be used or generated according to the present invention using the target molecule, is based on a similar principle. The manufacture of spiegelmers is described in the international patent application WO 98/08856. Spiegelmers are L-nucleic acids, which means that they are composed of L-nucleotides rather than aptamers which are composed of D-nucleotides as aptamers are. Spiegelmers are characterized by the fact that they have a very high stability in biological system and, comparable to aptamers, specifically interact with the target molecule against which they are directed. In the purpose of generating spiegelmers, a heterogonous population of D-nucleic acids is created and this population is contacted with the optical antipode of the target molecule, in the present case for example with the D-enantiomer of the naturally occurring L-enantiomer of the protein kinase N beta. Subsequently, those D-nucleic acids are separated which do not interact with the optical antipode of the target molecule. However, those D-nucleic acids interacting with the optical antipode of the target molecule are separated, optionally determined and/or sequenced and subsequently the corresponding L-nucleic acids are synthesized based on the nucleic acid sequence information obtained from the D-nucleic acids. These L-nucleic acids which are identical in terms of sequence with the aforementioned D-nucleic acids interacting with the optical antipode of the target molecule, will specifically interact with the naturally occurring target molecule rather than with the optical antipode thereof. Similar to the method for the generation of aptamers it is also possible to repeat the various steps several times and thus to enrich those nucleic acids specifically interacting with the optical antipode of the target molecule.

Any of the agents of the present invention can be used for the manufacture of a medicament or a pharmaceutical composition. This applies also to the nucleic acid coding for such agent, the vector containing such coding nucleic acid, and the cell containing such coding nucleic acid. Any of the possible use disclosed herein in connection with the medicament of the present invention is also disclosed for any pharmaceutical composition, and vice versa. The pharmaceutically composition preferably contains a pharmaceutically acceptable carrier. Such pharmaceutically acceptable carriers are known to the ones skilled in the art. Typically, the carriers strongly depend on the mode of administration. For system or intravenous administration the carrier is preferably a solution and more preferably a buffer solution such as, e.g,. a physiological buffered salt solution.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, a- anomeric sugars, epimeric sugars such as arabinose, xyloses or lyxoses, pyranose sugars, furanose sugars, sedoheptuloses, acyclic analogs, and abasic nucleoside analogs such as methyl riboside. One or more phosphodiester linkage may be replaced by alternative linking groups. These alternative linking groups include, but are not limited to, embodiments wherein phosphate is replaced by P(O)S ("thioate"), P(S)S ("dithioate"), "(0)NR2 ("amidate"), P(O)R, P(O)OR', CO or CH2 ("formacetal"), in which each R or R' is independently H or substituted or unsubstituted alkyl (1-20 C.) optionally containing an ether (-0-) linkage, aryl, alkenyl, cycloalkyl, cycloalkenyl, or araldyl. Not all linkages in a polynucleotide need be identical. The preceding description applies to all polynucleotides referred to herein, including RNA and DNA.

In a preferred embodiment, the nucleic acid is operably linked with another nucleic acid, preferably a vector. A nucleic acid is "operably linked," as used herein, when it is placed into a functional relationship with another nucleic acid sequence. For example, DNA for a presequence or secretory leader is operably linked to DNA for a antibody if it is expressed as a preprotein that participates in the secretion of the antibody; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, an enhancer may not have to be contiguous. Linking is accomplished by ligation at convenient restriction sites. If such sites do not exist, synthetic oligonucleotide adaptors or linkers are used in accordance with conventional practice.

A vector as preferably used herein is a nucleic acid which allows for the replication and more preferably the expression of an insert nucleic acid. The various elements which have to be contained in such vector are known to the ones skilled in the art and, together with representative examples of such vectors, e.g. available at IBA, Göttingen or described in Maniatis (Sambrook, J., Fritsch, E.F., and Maniatis, T., in Molecular Cloning: A Laboratory Manual. Cold Spring Harbor Laboratory Press, NY, Vol. 1, 2, 3 (1989).).

In a further aspect the present invention is related to a method for the treatment of a subject in need of such treatment. In connection with said method, an agent or a pharmaceutical composition as disclosed herein, is used and preferably administered to said subject. More preferably the subject is a mammal and more preferably a human being.

In a still further embodiment the present invention is related to a kit. Such kit preferably contains one or several of the agents disclosed herein. In a further embodiment, the kit contains an instruction leaflet. Preferably the agent(s) is/are contained in said kit in a form which is ready to use. Ready to use as preferably used herein means that, particularly, the agent is measured for single administrations.

The invention is now further illustrated by the following figures and examples from which further features, embodiments and advantages may be taken. More specifically,
- Fig.1: is illustrating the binding characteristics of the agent in accordance with the present invention, whereby in Figs 1A and C the binding of FcgammaRIIb blocking antibodies on general immune cells e.g. Macrophages (A) and B-cells (C) is illustrated and in Fig. 1B and D the improved characteristics of not blocking antibodies, which are the subject of the current invention, are depicted.
- Fig. 2: shows the clinical score in murine EAE in SJL/j mice which is a model for human multiple sclerosis upon application of different amounts of the antibody anti-Ly17.1 as a function of time.
- Fig. 3: shows an amino acid sequence alignment of the extracellular domains of FcgammaRIIa (IIA) and FcgammaRIIb (IIB), whereby the amino acid residues which are different in IIA and IIB and are not involved in Fc-fragment binding are boxed and represent the preferred target site of the agents of the present invention.

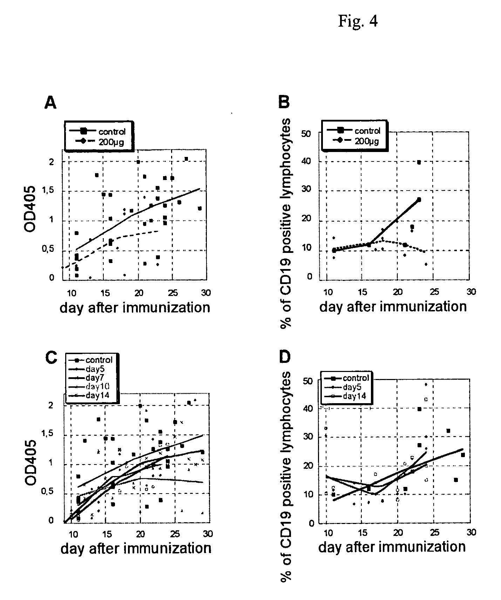

- Figs. 4A,C: are diagrams depicting the relative concentration of MOG-specific serum IgGs in the blood of treated (circles) versus untreated mice (squares) as a function of time, i.e. days after immunization, as detected by ELISA and as represented as OD 405, illustrating the influence of FcR antagonists and agonists on MOG-specific IgGs.
- Figs. 4B,D: are diagrams depicting the percentage CD19 expressing B cells from peripheral blood of control mice (squares) versus mice treated with 200µg sFcR or 50µg anti-FcgammaRII (Ly-17.1) antibody (circles) as a function of time, i.e. days after immunization, illustrating the influence of FcR antagonists and agonists on B cell count.

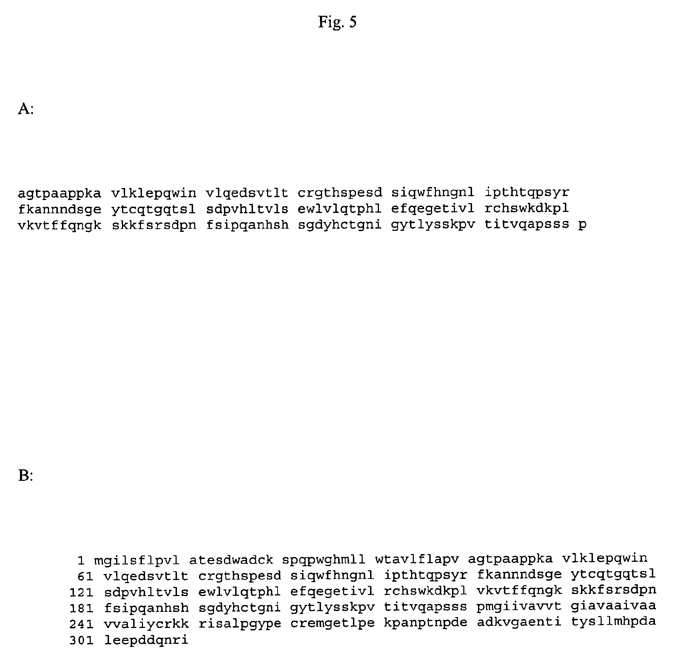

- Figs 5A,B: represent the amino acid sequences of the human FcgammaRIIb, whereby Fig. 5A depicts the N-terminal, extracellular domain corresponding to SEQ.ID.NO. 1, and Fig. 5B depicts the overall amino acid sequence of the human FcgammaRIIb corresponding to SEQ.ID.NO. 2 with the signal sequences being formed by amino acid residues 1 to 40, the extracellular domain being formed by amino acid residues 41 to 220, whereby the first, i. e. N-terminal, IgG domain is formed by amino acid residues 41 to 124, the second, i. e. C-terminal IgG domain is formed by amino acid residues 125 to 211 and the linker is formed by amino acid residues 212 to 220, followed by the transmembrane and intracellular region formed by amino acid residues 221 to 310.

### Example 1: Materials and Methods

If not indicated to the contrary, the following materials and methods were used in connection with the examples described herein.

### Animals.

Female SJL/j mice were purchased from Charles River Wiga and were immunized between 10 and 12 weeks of age. Female C57BL/6 mice were bred at the animal facility of the Max-Planck Institute of Biochemistry.

All animal experiments were performed according to the Bavarian state regulations for animal experimentation, and were approved by the responsible authorities.

### Induction of EAE.

SJL/j mice were injected subcutaneously in the back with 50µl of an emulsion containing equal amounts of 100µg recombinant rat MOG in PBS and CFA (Sigma) with Mycobacterium tuberculosis H37Ra (5mg/ml; Difco). Additionally 250ng pertussis toxin (List Laboratories) was injected intraperitoneally (i.p.) on the day of and two days following the immunization. Animals were weighed and inspected for disease severity on a daily basis.

Clinical scoring was performed as follows: 0=normal; 1= paralysis of the tail; 2= complete paralysis of the tail and partial paralysis of hind limbs; 3= complete paralysis of the tail and the hind limbs; 4= tetraplegia; and 5=death. I.P. treatment with soluble FcγRII or anti-FγRII was as indicated for individual experiments. Treated animals were taken randomly from individual cages to receive either treatment.

### Therapeutic protein and monoclonal antibody.

Recombinant soluble human FcRIIb was expressed in E.coli and prepared according to a standard protocol (Sondermann and Jacob 1999).

The K75.325 Hybridoma cell line is producing mouse antibodies detecting the mouse Ly-17.1 alloantigen of FcγRII. Cellular supernatant containing the antibody was collected and purified by a Protein A column (Biosepra) and subsequent gelfiltration (S200, Amersham).

### Detection of MOG-Autoantibodies by Enzyme-linked immunosorbent assay ELISA.

Serial blood samples were collected from the vena saphena as described previously (Hem et al. 1998). ELISA was performed with polystyrene 96-well PVC plates (NUNC) coated with 10µg/ml recombinant native MOG prepared according to a standard protocol (3) (1 h, 30°C) in 50 mM carbonate-bicarbonate buffer, pH 9.6. The plates were washed with PBS/0.05% Tween 20 (Sigma) and blocked with 5% BSA in PBS (pH 7.4) overnight at 4°C. After washing with PBS-Tween, 100 µl of plasma samples diluted 1:500 in PBS were incubated for 4 h at 37°C. Anti-MOG levels were determined directly using 100 µl peroxidase-conjugated mouse IgG-specific goat Ab (1:3000) (Amersham). All plates were developed with o-phenylenediamine dihydrochloride (Sigma), the reaction was stopped with 4 M H₂SO₄, and optical density was determined at 490 nm.

### Flow-cytometry analysis of peripheral blood.

For FACS analysis mice were bled with heparinized capillaries (Sarstedt). Red blood cells were lysed by incubation in 0.165 M NH₄Cl for 10 min. Cells were washed with PBS/1% BSA and stained with the anti-CD19-Cy3 conjugate antibody (PharMingen). Cells were analyzed with a FACScan® (Becton Dickinson). Appropriate analysis gates were used to enumerate total lymphocytes and B cells. A vial with cells stained with isotopic controls for IgG1-FITC was used as negative control. A positive signal was defined as being greater than non-specific background staining and analyses were considered informative when adequate numbers of events (10000) were collected in the lymphocyte gate. Data were analysed with CellQuest software (Becton Dickinson).

### sFcgammaR antagonist in EAE

Highly pure and homogeneous preparations of the soluble form of the ecto-domain of human FcgRIIb (Sondermann and Jacob 1999) which is also referred to as sFcgammaRIIB, was expected to compete with cell-standing FcRs for IC binding, thereby preventing stimulation of the immune cells upon immune complex recognition and consequently to ameliorate the course of the EAE.

### Example 2: Effect of anti-Ly17.1 antibody on EAE

EAE which was induced as described above in example 1 in SJL/j mice, was treated using Ly17.1 which is an antibody having the characteristics of the agents used in the prior art such as WO 2004/16750 and WO 2005/051999, for the treatment of autoimmune diseases.

The result is depicted in Fig. 2. Due do the mechanism described herein, the use of anti-Ly17.1 within a small concentration window only, namely 25 or 50 µg, was suitable to provide for a beneficial reaction as expressed by a comparatively reduced clinical score of the disease.

### Example 3: The influence of sFcgammaRIIb and anti-Ly-17.1 antibody on B cell count and the amount of MOG autoantibodies

Whereas the importance of T-cells in MS is well established, the contribution of the humoral immune response has recently gained increased attention. In neuropathological studies B-cells and antibodies have been detected in the CSF and in lesions of MS patients indicating their involvement in the pathogenesis of MS (Kanter et al. 2006, Ziemssen et al. 2005). Furthermore, the microenvironment of inflammatory sites in the CNS contains antigen presenting cells such as microglia and perivascular macrophages which activate T-cells and release cytokines such as IL6, BlyS and INF-gamma which favour the activation of B cells (McGeatchy et al. 2005, Rathmell 2004, Imi et al. 2005). The number of B cells is reported to increase during lesional activity and at later stages of the disease.

In mice models it could be demonstrated that EAE was accelerated and the disease severity elevated in the presence of pathogenic B cells and MOG-specific auto-antibodies (Ziemssen et al. 2005). To elucidate the influence of FcgammaR antagonists and agonists on the amount of auto-antibody producing B cells we induced EAE in SJL/j mice by immunization with recombinant rat MOG as described by Devaux *et al.* (Devaux et al 1997). MOG is a quantitatively minor component of CNS myelin but preferentially located at the accessible outermost lamellae of the myelin sheath, which supports the assumption that antibodies against the native MOG are important drivers of the EAE-model (Zhang et al. 2004). In order to quantify these presumably pathogenic antibodies we analyzed serum samples taken at various time points of the disease course by ELISA using recombinant refolded MOG as antigen (Breithaupt et al. 2003).

The concentration of conformational dependant anti-MOG antibodies increased during the course of the disease (Fig. 4A, C). Mice receiving sFcgammaRIIb treatment starting on day 14 showed stagnation in the increase of the auto-antibody titer in contrast to control mice (Fig. 4A). To confirm these results we determined the number of peripheral B cells using an anti- CD19 antibody in flow cytometry (FACS). Whole blood samples taken at different time points of the disease reveal that the number of B cells increased during the disease course in accordance with the anti-MOG antibody titer (Fig. 4B). After administration of sFcgammaRIIb, however no further increase in the CD19 positive B-cell population is observed indicating that the sFcgammaRIIb acts as a suppressor of B cell proliferation.

In analogy we analysed the serum of mice treated with the anti-Ly-17.1 antibody. Although the treatment showed a clear therapeutic effect, in contrast to mice treated with sFcgamma RIIb no significant decrease in the auto-antibody titer could be confirmed (Fig. 4C). Accordingly, the flow cytometry analysis of B cells in whole blood of mice treated with anti-Ly-17.1 did not reveal any influence on the amount of CD 19 positive B cells (Fig. 4D).

This experiment showing that the antibody level and in particular the CD19 positive B cells are not decreased by the treatment using the anti-Ly.17.1 antibody, is thus supportive for the rational underlying the technical teaching of the present invention. Furthermore, if animal are treated with increasing concentrations of the anti-Ly17.1 antibody an initial increase of the therapeutic effect is observed (administration of 10, 25 and 50 µg) but a spontaneous decrease of the therapeutic efficacy is seen when the concentration of the anti-Ly17.1 antibody is further increased to 75 µg per injection (Fig. 2). Thus, the administration of higher amounts of anti-Ly 17.1 leads to a reversal of the therapeutic effect and limits the usefulness of blocking anti FcgammaRIIb antibodies.

### Literature

The following list provides the bibliographic details of the various papers recited herein in a truncated manner and the disclosure of said papers is incorporated herein in its entirety by reference.

Abdul-Majid, K.B., A. Stefferl, C. Bourquin, H. Lassmann, C. Linington, T. Olsson, S. Kleinau, and R.A. Harris. 2002. Fc receptors are critical for autoimmune inflammatory damage to the central nervous system in experimental autoimmune encephalomyelitis. Scand J Immunol 55:70-81.

Breithaupt, C., A. Schubart, H. Zander, A. Skerra, R. Huber, C. Linington, and U. Jacob. 2003. Structural insights into the antigenicity of myelin oligodendrocyte glycoprotein. Proc Natl Acad Sci U S A 100:9446-9451. Epub 2003 Jul 9421.

Clynes, R., J.S. Maizes, R. Guinamard, M. Ono, T. Takai, and J.V. Ravetch. 1999. Modulation of immune complex-induced inflammation in vivo by the coordinate expression of activation and inhibitory Fc receptors. J Exp Med. 189:179-185.

Devaux, B., F. Enderlin, B. Wallner, and D.E. Smilek. 1997. Induction of EAE in mice with recombinant human MOG, and treatment of EAE with a MOG peptide. J Neuroimmunol. 75:169-173.

Fridman, W.H. 1993. Regulation of B-cell activation and antigen presentation by Fc receptors. Curr Opin Immunol 5:355-360.

Gessner, J.E., H. Heiken, A. Tamm, and R.E. Schmidt. 1998. The IgG Fc receptor family. Ann Hematol 76:231-248.

Hem, A., A.J. Smith, and P. Solberg. 1998. Saphenous vein puncture for blood sampling of the mouse, rat, hamster, gerbil, guinea pig, ferret and mink. Lab Anim 32:364-368.

Hibbs, M.L., P.M. Hogarth, and I.F. McKenzie. 1985. The mouse Ly-17 locus identifies a polymorphism of the Fc receptor. Immunogenetics 22:335-348.

Hogarth, P.M. 2002. Fc receptors are major mediators of antibody based inflammation in autoimmunity. Curr Opin Immunol. 14:798-802.

Imitola, J., T. Chitnis, and S.J. Khoury. 2005. Cytokines in multiple sclerosis: from bench to bedside. Pharmacol Ther. 106:163-177. Epub 2005 Jan 2011

Kanter, J.L., S. Narayana, P.P. Ho, I. Catz, K.G. Warren, R.A. Sobel, L. Steinman, and W.H. Robinson. 2006. Lipid microarrays identify key mediators of autoimmune brain inflammation. Nat Med. 12:138-143. Epub 2005 Dec 2011.

van Lent, P.L., K. Nabbe, A.B. Blom, A.E. Holthuysen, A. Sloetjes, L.B. van de Putte, S. Verbeek, and W.B. van den Berg. 2001. Role of activatory Fc gamma RI and Fc gamma RIII and inhibitory Fc gamma RII in inflammation and cartilage destruction during experimental antigen-induced arthritis. Am J Pathol. 159:2309-2320.

McGeachy, M.J., and S.M. Anderton. 2005. Cytokines in the induction and resolution of experimental autoimmune encephalomyelitis. Cytokine. 32:81-84. Epub 2005 Sep 2008.

Nakamura, A., K. Akiyama, and T. Takai. 2005. Fc receptor targeting in the treatment of allergy, autoimmune diseases and cancer. Expert Opin Ther Targets 9:169-190.

Rathmell, J.C. 2004. B-cell homeostasis: digital survival or analog growth? Immunol Rev. 197:116-128.

Ravetch, J.V., and S. Bolland. 2001. IgG Fc receptors. Annu Rev Immunol 19:275-290.

Samuelsson, A., T.L. Towers, and J.V. Ravetch. 2001. Anti-inflammatory activity of IVIG mediated through the inhibitory Fc receptor. Science. 291:484-486.

Slingsby, J.H., M.B. Hogarth, M.J. Walport, and B.J. Morley. 1997. Polymorphism in the Ly-17 alloantigenic system of the mouse FcgRII gene. Immunogenetics 46:361-362.

Sondermann, P., and U. Jacob. 1999. Human Fcgamma receptor IIb expressed in Escherichia coli reveals IgG binding capability. Biol Chem 380:717-721.

Sondermann, P., R. Huber, V. Oosthuizen, and U. Jacob. 2000. The 3.2-A crystal structure of the human IgG1 Fc fragment-Fc gammaRIII complex. Nature. 406:267-273.

Zhang, G.X., S. Yu, B. Gran, J. Li, D. Calida, E. Ventura, X. Chen, and A. Rostami. 2004. T cell and antibody responses in remitting-relapsing experimental autoimmune encephalomyelitis in (C57BL/6 x SJL) F1 mice. J Neuroimmunol 148: 1 -10.

Ziemssen, T., and F. Ziemssen. 2005. The role of the humoral immune system in multiple sclerosis (MS) and its animal model experimental autoimmune encephalomyelitis (EAE). Autoimmun Rev 4:460-467.

The features of the present invention disclosed in the specification, the claims and/or the drawings may both separately and in any combination thereof be material for realising the invention in various forms thereof.

## Claims

1. An agent that binds FcgammaRIIb with higher affinity than FcgammaRIIa but does not interfere with the function of the FcgammaRIIb to bind to the Fc-fragment of immune globulins.

2. The agent of claim 1 that specifically binds FcgammaRIIb, whereby the binding of immune complexes to FcgammaRIIB is not inhibited.

3. The agent of any of claims 1 to 2, whereby the binding site of the agent recognizes at least one amino acid residue that is different between FcgammaRIIa and Fcgamma RIIB, preferably two, more preferably three of such amino acid residues, whereby such amino acid(s) is/are not involved in Fc-fragment binding.

4. The agent according to claim 3, whereby such amino acid(s) is/are present in the N-terminal region or the N-terminal domain or the C-terminal linker region of FcgammaRIIb.

5. The agent according to any of claims 3 or 4, whereby the amino acids are selected from the group comprising amino acids 17, 32, 33, 35, 109 and 176 of the amino acid sequence of SEQ.ID.No 1, more preferably of human FcgammaRIIb.

6. The agent according to any of claims 3 to 5, whereby the amino acids are amino acids 32 to 35 of SEQ.ID.No.1.

7. The agent according to any of claims 1 to 6, wherein the binding affinity to FcgammaRIIb is higher than the binding affinity to FcgammaRIIa, preferably at least 2fold, more preferably at least 4fold, more preferably at least 10fold, more preferably at least 100fold, more preferably at least 1000fold, more preferably at least 10.000fold and most preferably 100.000 fold higher.

8. The agent according to any of claims 1 to 7, wherein the agent is selected from the group comprising antibodies, bispecific antibodies, trispecific antibodies, anticalines, aptamers and spiegelmers.

9. The agent according to claim 8, wherein the agent is an antibody, preferably a monoclonal antibody and more preferably a humanized antibody.

10. The agent according to claim 8 or 9, wherein the agent is a human antibody.

11. The agent according to any of claims 8 to 10, wherein the agent is a F(ab')₂ fragment.

12. The agent according to any of claims 8 to 10, wherein the agent is a F(ab) fragment.

13. The agent according to any of claims 8 to 10, wherein the agent is a single chain antibody.

14. The agent according to any of claims 8 to 13, wherein the agent is an antibody of the IgG class.

15. The agent according to any of claims 8 to 13, wherein the agent is an antibody of the IgE type.

16. A nucleic acid coding the agent and more preferably an antibody according to any of claims 1 to 15.

17. A vector, preferably an expression vector, comprising a nucleic acid according to claim 16.

18. A cell, preferably an isolated cell, comprising a nucleic acid according to claim 16 or a vector according to claim 17.

19. A pharmaceutical composition comprising an agent, preferably an antibody or a nucleic acid according to any of the preceding claims, whereby the composition preferably contains a pharmaceutically acceptable vehicle.

20. Use of an agent, preferably an antibody, according to any of the preceding claims for the manufacture of a medicament.

21. Use of a nucleic acid according to any of the preceding claims for the manufacture of a medicament.

22. Use according to claim 20 or 21, whereby the medicament is for the treatment of an autoimmune disease.

23. Use according to claim 20 or 21, whereby the medicament is for the treatment of a B-cell lymphoma.

24. Use according to claim 22, whereby the autoimmune disease is selected from the group comprising inflammatory responses such as inflammatory skin diseases including psoriasis and dermatitis inclduing atopic dermatitis; systemic scleroderma and sclerosis; responses associated with inflammatory bowel disease including Crohn's disease and ulcerative colitis; respiratory distress syndrome including adult respiratory distress syndrome; ARDS; dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus including Type I diabetes mellitus or insulin dependent diabetes mellitis; multiple sclerosis; Reynaud's syndrome; autoimmune thyroiditis; allergic encephalomyelitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious anemia (Addison's disease); diseases involving leukocyte diapedesis; central nervous system (CNS) inflammatory disorder; multiple organ injury syndrome; hemolytic anemia including cryoglobinemia or Coombs positive anemia ; myasthenia gravis; antigen-antibody complex mediated diseases; anti-glomerular basement membrane disease; antiphospholipid syndrome; allergic neuritis; Graves' disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) and autoimmune thrombocytopenia.

25. Use according to any of claims 20 to 24, wherein the agent is an antibody of the IgG type as defined in any of the proceeding claims.

26. Use according to any of claims 20 to 24, wherein the disease is selected from the group comprising allergic diseases and asthma.

27. Use according to claim 26, wherein the agent is an antibody of the IgE type.

28. A method for producing an antibody that specifically binds FcgammaRIIb but does not block a function of the FcgammaRIIb, more preferably of an antibody as described in any of claims 1 to 15, whereby the method comprises the following steps:
(a) immunizing a mouse with a peptide containing amino acids 32-35 (RGTH) of FcgammaRIIb;
(b) optionally booster immunizing said mouse for a time sufficient to elicit an immune response;
(c) producing hybridoma cell lines from spleen cells of said mouse; and
(d) screening said hybridoma cell lines for one or more hybridoma cell lines that produce antibodies that bind FcgammaRIIb but does not block a function of the FcgammaRIIb.
